(19) Europäisches Patentamt
European Patent Office
Office européen des brevets

(11) Publication number: **0 526 951 A1**

# (12) EUROPEAN PATENT APPLICATION

(21) Application number: **92202438.5**

(22) Date of filing: **06.08.92**

(51) Int. Cl.$^5$: **C07D 333/54**, C07D 333/64, C07C 323/22, A01N 43/12

(30) Priority: **07.08.91 GB 9117053**

(43) Date of publication of application: **10.02.93 Bulletin 93/06**

(84) Designated Contracting States: **AT BE CH DE DK ES FR GB GR IT LI LU NL PT SE**

(71) Applicant: **SHELL INTERNATIONALE RESEARCH MAATSCHAPPIJ B.V.**
**Carel van Bylandtlaan 30**
**NL-2596 HR Den Haag(NL)**

(72) Inventor: **Gilkerson, Terence**
**11 The Ness**
**Canterbury, Kent CT1 2NL(GB)**
Inventor: **Gilmore, Ian James**
**32 Waterloo Road**
**Sittingbourne, Kent ME10 2LN(GB)**

## (54) 3-Phenyl benzo b thiophenes as pesticidal compounds.

(57) Compounds of the general formula

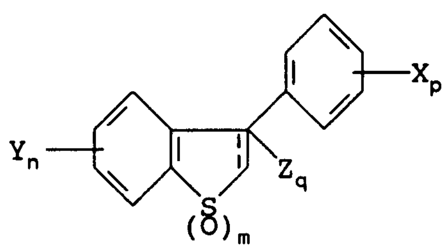

I

in which

X and Y each represents halogen, alkyl, haloalkyl, alkoxy, haloalkoxy, alkylenedioxy, alkylthio, halo-substituted alkylthio, alkylsulphinyl, halo-substituted alkylsulphinyl, alkylsulphonyl, halo-substituted alkylsulphonyl, cyano, nitro, amino, alkylamino, dialkylamino, acetamido, N-alkylacetamido, optionally substituted benzyl, optionally substituted benzoyl, optionally substituted phenylthio, optionally substituted phenylsulphinyl, optionally substituted phenylsulphonyl or optionally substituted phenoxy radical,

p is 0 to 5,
n is 0 to 4,
m is 0, 1 or 2,
Z represents hydrogen, hydroxy or OR where R represents acyl,
q is 0 or 1, and
⁞| represents a single bond (when q is 1) or a double bond (when q is 0), have useful pesticidal properties.

EP 0 526 951 A1

The present invention relates to benzo[b]thiophenes, processes for their preparation, benzo[b]-thiophene-containing compositions and the use of such compounds and compositions as pesticides, especially acaricides.

US Patent 3433874 discloses inter alia 2-chloro-3-phenylbenzothiophene compounds having pesticidal activity.

The applicants have now discovered a different group of benzo[b]thiophene compounds which have good acaricidal, particularly ovicidal, properties.

Accordingly, the present invention provides a pesticidal composition which comprises a compound of the general formula

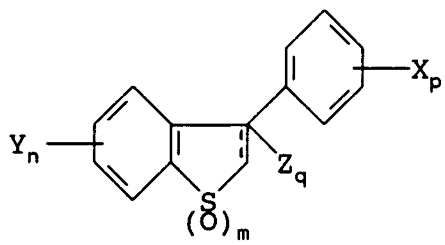

I

in which

X and Y each represents a halogen atom or an alkyl, haloalkyl, alkoxy, haloalkoxy, alkylenedioxy, alkylthio, halo-substituted alkylthio, alkylsulphinyl, halo-substituted alkylsulphinyl, alkylsulphonyl, halo-substituted alkylsulphonyl, cyano, nitro, amino, alkylamino, dialkylamino, acetamido, N-alkyl-acetamido, optionally substituted benzyl, optionally substituted benzoyl, optionally substituted phenylthio, optionally substituted phenylsulphinyl, optionally substituted phenylsulphonyl or optionally substituted phenoxy radical,

p represents 0 or an integer of from 1 to 5,

n represents 0 or an integer of from 1 to 4, and any two or more substituents represented by X and/or Y may be the same or different,

m represents 0, 1 or 2,

Z represents a hydrogen atom, a hydroxy group or a radical OR where R represents an acyl moiety,

q represents 0 or 1, and

⁝| represents a single bond (when q is 1) or a double bond (when q is 0),

provided that if q represents 1 and Z represents a hydroxy group or an OR radical, then $n = 0$ and $p = 0$, or when $n \geqq 1$, then $p = 0$, or when $p \geqq 1$, then $n = 0$, together with a suitable carrier.

The present invention also provides a method of combating pests at a locus, which comprises applying to the locus a compound of the general formula I specified above or a pesticidal composition according to the invention containing a compound of the general formula I.

The pesticidal use contemplated includes, not only acaricidal, but also insecticidal, use.

Certain of the compounds of the invention, and in particular those compounds in which Z is OH, also form useful precursors for pesticidally active compounds of the general formula I.

Except where otherwise stated, throughout this Specification, including claims, an alkyl or alkylene radical or moiety has up to 6 carbon atoms, preferably has up to 3 carbon atoms. A preferred alkyl radical or moiety is methyl, and a preferred alkylene moiety is methylene.

A phenyl moiety in a radical represented by X and/or Y may be unsubstituted or substituted by one or more of the same or different substituents. Suitable substituents are, for example, halogen atoms and alkyl, alkoxy, haloalkyl, cyano and nitro radicals.

If a halo-substituted alkyl, alkoxy, alkylthio, alkylsulphinyl or alkylsulphonyl group contains two or more halogen atoms, these may be the same or different. Two alkyl moieties in a dialkylamino radical represented by X and/or Y may also be the same or different.

Halogen atoms may be fluorine, chlorine, bromine or iodine atoms, with fluorine and, more especially, chlorine being preferred; a preferred haloalkyl radical is trifluoromethyl.

The benzene ring of the benzothiophene ring structure and the phenyl ring in the 3-position of that structure may each be unsubstituted or substituted, and the number and identity of substituents in each of those rings may be the same or different.

When p is greater than 1, two or more of the substituents X present may be the same or different. When n is greater than 1, two or more of the substituents Y present may be the same or different.

An acyl moiety in an OR radical represented by Z may be, for example, an alkanoyl or a haloalkanoyl

moiety, preferably acetyl.

It should of course be understood that, where appropriate, compounds of the general formula I may be in the form of individual stereoisomers, free from other isomers, or mixed therewith in any proportion.

Some compounds of the general formula I are known in the literature:

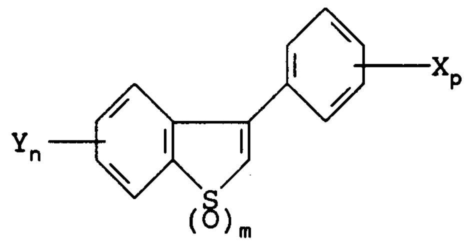

| $X_p$ | $Y_n$ | m | CAS Registry Number or Chemical Abstracts (CA) Reference |
|---|---|---|---|
| - | - | 0 | 14315-12-9 |
| - | - | 1 | 70445-87-3 |
| - | - | 2 | 27183-55-7 |
| - | 6-OMe | 0 | 63762-92-5 |
| - | 7-Me | 0 | 33428-16-9 |
| - | 2-Me | 2 | CA:63/16286 |
| - | 5,7-diMe | 0 | CA:64/3452 |
| o-Br | - | 0 | 24257-27-0 |
| m-Br | - | 0 | 24257-28-1 |
| p-Br | - | 0 | 24257-29-2 |
| p-Br | 7-Me | 0 | 31493-46-6 |
| p-Br | 5-Me | 0 | 38251-56-8 |
| p-Cl | 7-Me | 0 | 27123-71-3 |
| p-Cl | 2,6-diCl | 1 | 39561-70-1 |
| p-F | 7-Me | 0 | 30089-61-3 |
| o-MeO | - | 0 | 24257-22-5 |
| m-MeO | - | 0 | 24257-21-4 |
| p-MeO | - | 0 | 24257-23-6 |
| - | 7-MeO | 0 | 30089-55-5 |
| - | 4,7-diMeO | 0 | 30089-61-3 |
| - | 5-Br | 0 | 30160-87-3 |
| - | 5-Cl | 0 | 28540-36-5 |
| o-SMe | - | 0 | 59563-11-0 |

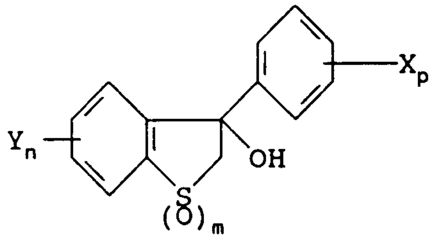

| $X_p$ | $Y_n$ | m | CAS Registry Number or Chemical Abstracts (CA) Reference |
|---|---|---|---|
| - | - | 0 | 28540-33-2 |
| - | - | 2 | 62521-50-0 |
| p-Me | - | 0 | 120563-04-4 |

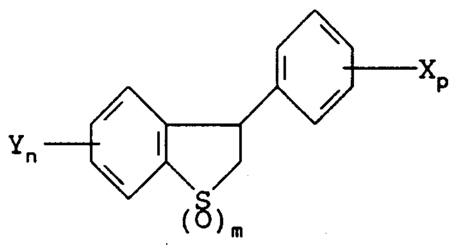

| $X_p$ | $Y_n$ | m | CAS Registry Number or Chemical Abstracts (CA) Reference |
|---|---|---|---|
| - | - | 0 | CA:65/18549 |
| - | - | 2 | CA:65/18549 |
| p-Me | - | 0 | 74881-91-7 |
| p-Me | - | 2 | 61503-23-9 |
| 2,5-diMe | - | 2 | 74881-96-2 |
| 3,4-diMe | - | 2 | 74881-94-0 |
| 3,4-diMe | - | 2 | 74881-95-1 |
| - | 5,7-diMe | 0 | CA:65/18549 |
| - | 5,7-diMe | 2 | CA:65/18549 |
| - | 5-Me | 0 | CA:65/18549 |
| - | 5-Me | 2 | CA:65/18549 |
| - | 5-Et | 0 | CA:65/18549 |
| - | 5-Et | 2 | CA:65/18549 |

However, the applicants are not aware of any prior disclosure of compounds of the general formula I for pesticidal or acaricidal use.

The invention also provides

(a) a 3-phenylbenzo[b]thiophene or oxide thereof of the general formula

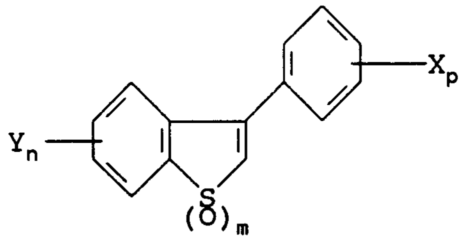

Ia

in which X, Y, p, n and m have the meanings given above and n + p ≧ 1, provided that: if, simultaneously, m = 0, n = 0 and p = 1, then X is not a bromine atom or a methoxy group or 2-methylthio; if, simultaneously, m = 0 and p = 0, then $Y_n$ is not 5-bromo, 5-chloro, 6- or 7-methoxy, 7-methyl, 5,7-dimethyl or 4,7-dimethoxy; if simultaneously, m = 0, n = 1 and p = 1, then X is not 4-bromo when Y is 5-methyl, nor any 4-halo when Y is 7-methyl; if simultaneously m = 1 and p = 1, then X is not 4-chloro when $Y_n$ is 2,6-dichloro; and if simultaneously m = 2, p = 0 and n = 1, then Y is not 2-methyl.

(b) a 3-phenyl-2,3-dihydrobenzo[b]thiophene or oxide thereof of the general formula

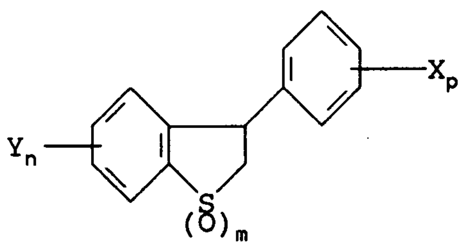

Ib

in which X, Y, p, n and m have the meanings given above, provided that: if, simultaneously, m = 0 or 2 and n = 0, then p ≧ 1 and additionally $X_p$ is not 4-methyl or (when m = 2) 2,5- or 3,4-dimethyl; and if, simultaneously, m = 0 or 2 and p = 0, then n ≧ 1 and additionally $Y_n$ is not 5-methyl, 5-ethyl or 5,7-dimethyl;

(c) a 3-hydroxy-3-phenyl-2,3-dihydrobenzo[b]thiophene or derivative thereof or oxide of these of the general formula

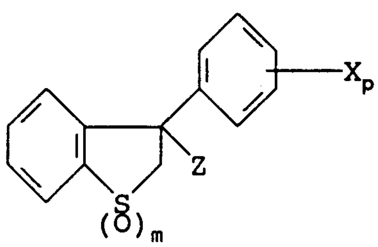

Ic

in which X, p and m have the meanings given above, and Z represents a hydroxy group or an OR group, provided that: if Z represents a hydroxy group, and if, simultaneously, m = 0 or 2, then p ≧ 1; and if, simultaneously, m = 0 and p = 1, then X is not 4-methyl;

and

(d) a 3-hydroxy-3-phenyl-2,3-dihydrobenzo[b]thiophene or derivative thereof or oxide of these of the general formula

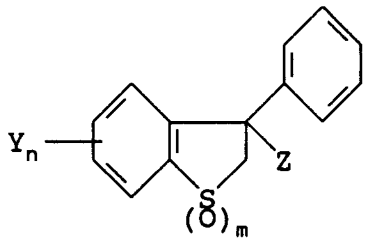

Id

in which Y, n and m have the meanings given above, and z represents a hydroxy group or an OR group as hereinbefore defined.

Compounds of the general formula I may be prepared by methods known per se.

A compound of the general formula Ic in which Z represents a hydroxy group may be prepared, for example, by reacting thioanisole of the formula

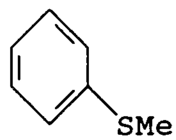

II

with an alkyl lithium compound, preferably n-butyl lithium, and an optionally substituted benzoyl halide of the general formula

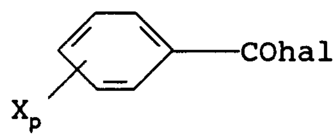

III

in which X and p have the meanings given above, to form a compound of the general formula

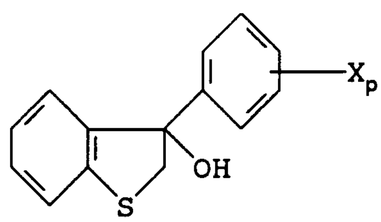

$Ic_1$

in which X and p have the meanings given above, which may then, if desired, be oxidised to a compound of the general formula

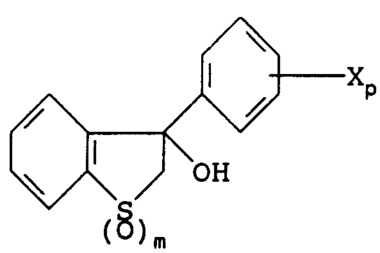

$Ic_2$

in which X and p have the meanings given above and m represents 1 or 2.

The reaction between compounds of the formulae II and III in the presence of an alkyl lithium compound is suitably carried out in two stages, the first stage being the reaction between thioanisole and the alkyl lithium compound in the presence of a base such as, for example, tetramethylenediamine, at reduced temperature, suitably cooled to -10 °C, in a suitable solvent such as, for example, anhydrous ether; this is followed by reaction with the benzoyl halide, for example the chloride, at reduced temperature, for example at -80 °C. Further details are given, for example in Synthesis (1988), pages 888-890.

A compound of the general formula Id in which Z represents a hydroxy group, may be prepared by cyclising in the presence of a base and of a suitable solvent, a compound of the general formula

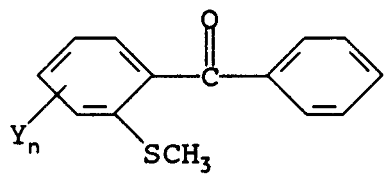

IV

in which Y and n have the meanings given above, to form a compound of the general formula

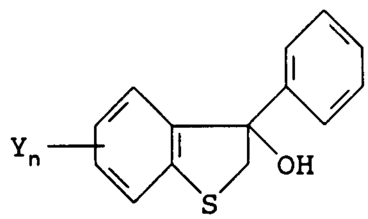

$Id_1$

in which Y and n have the meanings given above, which may also be oxidized, if desired, to the corresponding 1-oxide or 1,1-dioxide.

The base utilized in such a reaction is suitably an alkali metal hydride or alkoxide; sodium and potassium hydrides and alkoxides are very suitable; sodium hydride is preferred. The solvent is conveniently an alcohol or any inert polar solvent such as dimethylformamide or dimethyl sulphoxide; dimethylformamide, especially dry dimethylformamide, is preferred.

Suitably the reaction is carried out at elevated temperature, usefully at the reflux temperature of the reaction medium. Usual reaction times are of the order of 2 to 10 hours, for example 5 hours.

Conversion of a 3-hydroxy-2,3-dihydro benzothiophene compound of the general formula $Ic_1$ or $Id_1$ to the corresponding 1-oxide or 1,1-dioxide may be carried out, for example, with a suitable oxidising agent, such as, for example, with m-chloroperoxybenzoic acid in a suitable solvent, for example, in methylene chloride, or with hydrogen peroxide in glacial acetic acid. The reaction is usually carried out at room temperature for a period of 1 to 16 hours, for example 12 hours. Preferably the molar ratio of the oxidising agent to the thiophene is substantially 1.1:1 for sulphoxide formation and 2.2:1 for sulphone formation.

A compound of the general formula Ic or Id in which Z represents OR may be prepared from the corresponding hydroxy compound by reaction with the appropriate anhydride or halide, for example an alkanoyl or haloalkanoyl anhydride or halide. In the case of the alkanoyl or haloalkanoyl halide the use of a suitable base, such as, for example triethylamine, is required. For preparation of a compound of the general formula Ic or Id in which Z represents OR, and m = 1 or 2, oxidation may be carried out before or after acylation.

A compound of the general formula Ia may be prepared, for example,

(i) from a compound of the general formula $Ic_1$ given above by reaction with a dehydrating agent such as, for example, p-toluenesulphonic acid, glacial acetic acid or concentrated sulphuric acid, to give a compound of the general formula

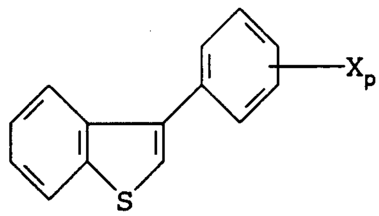

Ia$_1$

in which X and p have the meanings given above, which may then, if desired, be oxidized to give a compound of the general formula

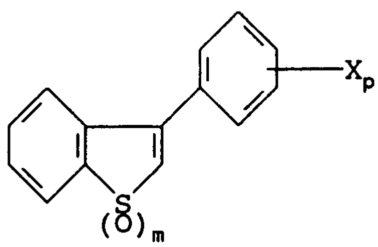

Ia$_2$

in which X and p have the meanings given above and m represents 1 or 2,

(ii) from a compound of the general formula Id$_1$ given above by reaction with a dehydration agent, as in (i) above, to give a compound of the general formula

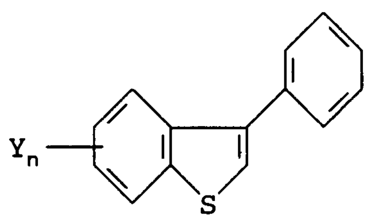

Ia$_3$

which may then, if desired, be oxidized to give a corresponding 1-oxide or 1,1-dioxide, or

(iii) from a compound of the general formula

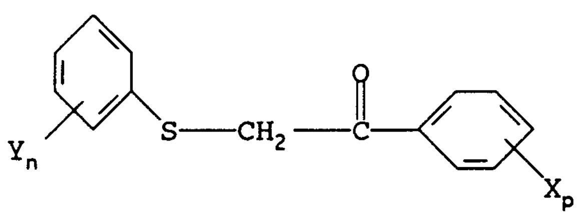

V

in which X, Y, p and n have the meanings given above, by cyclising in the presence of a dehydrating agent such as, for example, polyphosphoric acid or methanesulphonic acid, or from a compound of the general formula

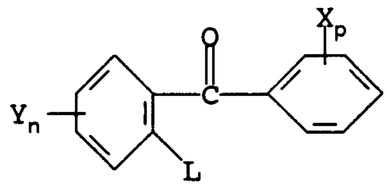

VI

in which X, Y, p and n have the meanings given above and L represents a leaving group, by reacting with thioglycolic acid in the presence of a base and a solvent, to form a compound of the general formula

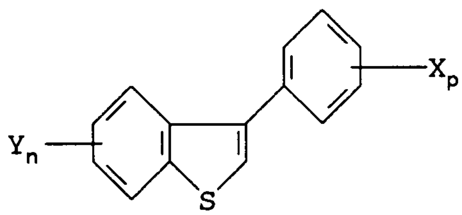

$Ia_4$

in which X, Y, p and n have the meanings given above, which may then, if desired, be oxidized to give a compound of the general formula

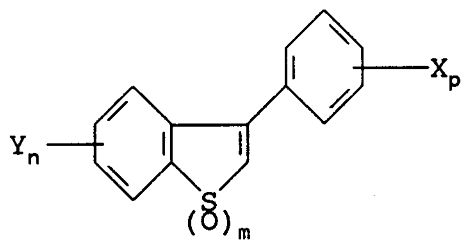

Ia

in which X, Y, p and n have the meanings given above and m represents 1 or 2.

The reaction between a compound of the formula $Ic_1$ or $Id_1$ and dehydrating agent may be carried out in a suitable solvent such as, for example, toluene. The reaction is usually carried out at reflux, for example for half an hour. Further details of the dehydration using p-toluenesulphonic acid are given, for example, in Synthesis (1988), pages 888-890.

The cyclisation of a compound of the general formula V may be carried out for example with polyphosphoric acid at a temperature in the range of from 100 to 180 ° C, for example at 100 ° C, suitably for a period of from 2 to 24 hours; alternatively, when methanesulphonic acid is used, the reaction is suitably carried out at a temperature in the range of from 10 to 50 ° C, preferably at 20 ° C, suitably for a period of from 2 to 20 hours.

A compound of the general formula V may be prepared, for example, by reaction of a compound of the general formula

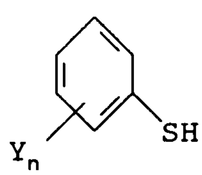

VII

in which Y and n have the meanings given above, with a suitable base and an optionally substituted phenacyl halide of the general formula

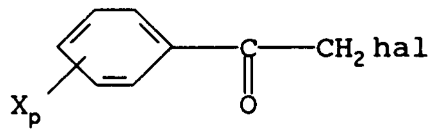

VIII

in which X and p have the meanings given above.

It should of course be understood that in compounds of the general formulae IV, V and VII at least one of the positions ortho to the side chain/SH group must be unsubstituted.

The base used for the reaction between compounds of the general formulae VII and VIII may be, for example, an alkali metal hydride, preferably sodium hydride, an alkali metal, preferably sodium, an alkali metal alkoxide, preferably a sodium alkoxide, such as, for example, the methoxide or ethoxide, an alkali metal hydroxide, for example sodium or potassium hydroxide, or an alkali metal carbonate, preferably potassium carbonate. Reaction is generally carried out in an appropriate solvent. Thus, for example, an alkali metal hydride may be used in ether, tetrahydrofuran or aromatic hydrocarbons, an alkali metal may be used in anhydrous ether, an alkali metal alkoxide or hydroxide in an alcohol, a hydrocarbon, dimethylformamide or dimethyl sulphoxide, and an alkali metal carbonate in dimethylformamide or dimethyl sulphoxide. The reaction is suitably carried out in two stages, the first stage being the reaction between the compound of the general formula VII and the base, which is suitably carried out at room temperature; this is followed by reaction with a phenacyl halide of the general formula VIII, preferably the bromide, suitably at an appropriate temperature in the range of from -5°C to 100°C.

A leaving group is any group that will, under the reaction conditions, cleave from the starting material thus promoting reaction at a specified site.

The leaving group, L, in a compound of general formula VI, is suitably a halogen atom or a nitro group, and is especially a fluorine atom.

The base used for the reaction of thioglycolic acid with a compound of general formula VI may be, for example, an alkali metal hydride, suitably sodium or potassium hydride, an alkali metal hydroxide, suitably sodium or potassium hydroxide, or an alkali metal carbonate, suitably sodium carbonate, and is especially potassium carbonate. The solvent used may be any inert polar solvent; dimethyl sulphoxide or dimethylformamide are particularly suitable, especially dimethylformamide. Suitably anhydrous or dried base and solvent are utilised.

The reaction of a compound of general formula VI with thioglycolic acid is suitably carried out at elevated temperature, preferably at the reflux temperature of the reaction medium. Usual reaction times are of the order of 15 to 20 hours.

The conversion of a compound of the general formula $Ia_1$, $Ia_3$ or $Ia_4$ to the corresponding 1-oxide or 1,1-dioxide may be carried out as described above for oxidation of a compound of the general formula $Ic_1$ or $Id_1$.

A compound of the general formula Ib may be prepared, for example,

(i) by reacting a compound of the general formula

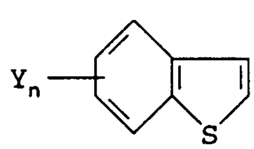

IX

in which Y and n have the meanings given above, in the presence of aluminium chloride with a compound of the general formula

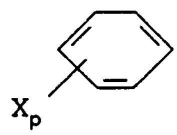

X

in which X and p have the meanings given above, and, if required, separating the desired isomer of the general formula

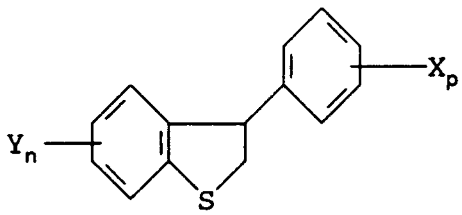

$Ib_1$

in which X, Y, p and n have the meanings given above, or

(ii) by reducing a compound of the general formula $Ia_4$ given above to give a compound of the general formula $Ib_1$ above,

and then, if desired, oxidising the compound of the general formula $Ib_1$ to form a compound of the general formula

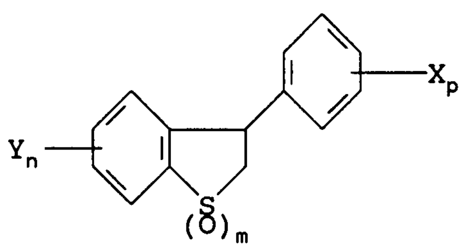

Ib

in which X, Y, p and n have the meanings given above and m represents 1 or 2.

The reaction between compounds of the general formulae IX and X may be carried out, for example, as described in J. Chem. Research Synopsis 1981 (10) p 307 ff.

Reduction of the compound of the general formula Ia may be carried out, for example, with sodium in ethanol or by catalytic hydrogenation.

To form a compound of formula Ib in which m is 1 or 2, from a compound of formula $Ib_1$ obtained via process variant (i) above, either the separated isomer $Ib_1$ may be oxidized directly, or the isomeric mixture containing isomer $Ib_1$ may be oxidised and the isomer Ib isolated thereafter. The oxidation to the corresponding 1-oxide or 1,1-dioxide may be carried out as described above for the oxidation of a compound of the general formula $Ic_1$ or $Id_1$.

If appropriate one or more other conversions of a compound of the general formula I into another such compound may be carried out.

Compounds of the general formula I may be isolated by conventional methods, for example the separation of isomers may be carried out by standard techniques.

Compounds of the formulae III, IV, VI, VII, VIII, IX and X are either known or can be prepared from known compounds by processes analogous to known processes. For example a compound of the formula IV may be prepared from a compound of formula VI (in which $p = 0$) by reaction with a sodium alkane thiol in dry dimethylformamide at reflux.

Compounds of the general formula V are novel and also form part of the subject of this invention; such compounds may be prepared by processes analogous to known processes.

Compounds of the general formula I exhibit pesticidal activity as is demonstrated, for example, by their action against eggs of the glasshouse red spider mite.

Accordingly, the invention also provides a compound of the general formula I for use as a pesticide. The invention further provides the use of a compound of the general formula I for the manufacture of an agent for use as a pesticide, and the use of such a compound as a pesticide. Generally, the agent is in the form of a composition comprising a compound of the general formula I and a suitable carrier.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating pesticidal compositions may be used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilizers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosene and light mineral oils; chlorinated hydrocarbons, for example carbon tetrachloride, perchloroethylene and trichloroethylene. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a compositon according to the invention is a surface-active agent. For example the composition may contain at least two carriers, at least one of which is a surface-active agent.

A surface-active agent may be an emulsifying agent, a dispersing agent or a wetting agent; it may be non-ionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The invention further provides a process for the preparation of a pesticidal composition which comprises bringing a compound of the general formula I, together with a carrier, into a form suitable for application as a pesticide.

An agent or composition of the invention may, for example, be formulated as a wettable powder, dust, granules, emulsifiable concentrate, suspension concentrate, solution, dispersion, emulsion or aerosol.

Wettable powders usually contain 25, 50 or 75% by weight of active ingredient and usually contain in addition to a solid inert carrier, 3-10% by weight of a dispersing agent and, where necessary, up to 10% by weight of stabilizer(s) and/or other additives such as penetrants or stickers. Dusts are usually formulated as a dust concentate having a similar composition to that of a wettable powder but without a dispersant, and are diluted in the field with further solid carrier to give a composition usually containing $\frac{1}{2}$ to 10% by weight of active ingredient. Granules are usually prepared to have a size between 10 and 100 BS mesh (1.676 - 0.152mm), and may be manufactured by agglomeration or impregnation techniques. Generally, granules will contain $\frac{1}{2}$ to 75% by weight of active ingredient and 0 to 10% by weight of additives such as stabilizers, surfactants, slow release modifiers and binding agents. The so-called "dry flowable powders" consist of relatively small granules having a relatively high concentration of active ingredient. Emulsifiable concentrates usually contain, in addition to a solvent and, where necessary, co-solvent, 10 to 50% w/v active ingredient, 2 to 20% w/v emulsifiers and 0 to 20% w/v of other additives such as stabilisers, penetrants and

corrosion inhibitors. Suspension concentrates are usually compounded so as to obtain a stable, non-sedimenting flowable product and usually contain 10 to 75% by weight active ingredient, 0.5 to 15% by weight of dispersing agents, 0.1 to 10% by weight of suspending agents such as protective colloids and thixotropic agents, 0 to 10% by weight of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

A composition of the invention may also contain one or more other ingredients, for example other compounds possessing pesticidal, herbicidal, or fungicidal properties.

The following Examples illustrate the invention.

Example 1

Preparation of 3-hydroxy-3-phenyl-2,3-dihydrobenzo[b]thiophene

A solution of n-butyl lithium (2.4M, 41ml) was added dropwise under dry nitrogen to a stirred solution of thioanisole (6.2g) and tetramethylenediamine (6.7g) in anhydrous diethyl ether (175 ml), cooled to -10°C. The mixture was allowed to come to room temperature and stirring was continued for a further 12 hours. The mixture was then cooled to -80°C and a solution of benzoyl chloride (6.8g) in dry diethyl ether (25ml) was added gradually. The resultant mixture was stirred for a further 1 hour at -80°C and then allowed to warm to room temperature. After stirring for a further 16 hours, the mixture was poured into water and the pH adjusted to 4-5 using 10% aqueous hydrochloric acid. The aqueous solution was extracted with diethyl ether (3 x 100 ml), dried over anhydrouz magnesium sulphate and evaporated. The residual oil was purified on a silica gel column using dichloromethane as eluant to give the title compound as a pale yellow viscous oil (2.5g).

| | | |
|---|---|---|
| Theory for $C_{14}H_{12}OS$<br>Found | C 73.7%<br>C 75.1% | H 5.3%<br>H 5.5% |
| m/e Theory: Found 228:228 | | |

Example 2

Preparation of 3-phenylbenzo[b]thiophene

3-hydroxy-3-phenyl-2,3-dihydrobenzo[b]thiophene (2.0g), prepared in Example 1, and p-toluenesulphonic acid (0.1g) in toluene (20ml) was refluxed for half an hour. The solution was poured onto water and the whole was extracted with diethyl ether (3 x 50ml). The ether extracts were dried over anhydrous magnesium sulphate and evaporated. The residual oil was chromatographed on a silica gel column using hexane as eluant to give the title compound as a colourless oil (1.5g).

| | | |
|---|---|---|
| Theory for $C_{14}H_{10}S$<br>Found | C 80.0%<br>C 81.6% | H 4.8%<br>H 5.1% |
| m/e Theory: Found 210:210 | | |

Example 3

Preparation of 3-phenylbenzo[b]thiophene-1,1-dioxide

3-phenylbenzo[b]thiophene (0.6g), prepared in Example 2, in dichloromethane (10ml) was treated with

m-chloroperoxybenzoic acid (1.0g) at ambient temperature and left to stir overnight. The reaction mixture was filtered, the methylene chloride evaporated and the residue purified on a silica gel column using dichloromethane as eluant to give the title compound (0.4g) as a white solid of m.p. 157-158 °C.

| | | |
|---|---|---|
| Theory for $C_{14}H_{10}SO_2$<br>Found | C 69.4%<br>C 69.9% | H 4.1%<br>H 4.0% |
| m/e Theory: Found 242:242 | | |

Example 4

(a) Preparation of 2-chloro-6-methylmercaptobenzophenone

2,6-dichlorobenzophenone (15g) and sodium methane thiol (4.8g) in dry dimethylformamide (300 ml) were refluxed for 2 hours. After cooling, the mixture was poured into water and the aqueous solution extracted with diethyl ether. The combined ether extracts were dried over magnesium sulphate, evaporated and the residue purified on a silica gel column using 25% v/v diethylether-hexane as eluant to give 2-chloro-6-methylmercaptobenzophenone (10.5g) as a white solid of melting point 82-83 °C.

| | | |
|---|---|---|
| Theory for $C_{14}H_{11}SOCl$<br>Found | C 64.1%<br>C 64.0% | H 4.2%<br>H 4.1% |
| m/e Theory: Found 262:262 | | |

(b) Preparation of 4-chloro-3-hydroxy-3-phenyl-2,3-dihydrobenzo[b]thiophene

2-chloro-6-methylmercaptobenzophenone (6.5g) in dry dimethylformamide (20ml) was added to a suspension of sodium hydride (1g, 60% disp. in oil) in dry dimethylformamide (70ml). The reaction mixture was refluxed for 5 hours, cooled, poured into water, acidified with dilute hydrochloric acid and extracted with diethyl ether. The combined ether extracts were dried over magnesium sulphate, evaporated and the residue purified on a silica gel column using methylene chloride as eluant to give 4-chloro-3-hydroxy-3-phenyl-2,3-dihydrobenzo[b]thiophene (2.8g) as a colourless oil.

| | | |
|---|---|---|
| Theory for $C_{14}H_{11}SOCl$<br>Found | C 64.1%<br>C 67.3% | H 4.2%<br>H 4.6% |
| m/e Theory: Found 262:262 | | |

Example 5

Preparation of 4-chloro-3-phenylbenzo[b]thiophene

4-chloro-3-hydroxy-3-phenyl-2,3-dihydrobenzo[b] thiophene (2.8g), prepared in Example 4, and p-toluene sulphonic acid (0.2g) in toluene were refluxed for 2 hours. The toluene was evaporated and the residue chromatographed on a silica gel column using methylene chloride as eluant to give 4-chloro-3-phenyl benzo[b]thiophene (1.4g) as a colourless oil.

| | | |
|---|---|---|
| Theory for $C_{14}H_9SCl$<br>Found | C 68.8%<br>C 68.9% | H 3.7%<br>H 3.7% |
| m/e Theory: Found 244:244 | | |

Example 6

(a) Preparation of p-tolyl phenacyl sulphide

p-thiocresol (12.4g) in dry diethyl ether (20ml) was added dropwise to a stirred suspension of sodium hydride (4g, 60% disp. in oil) in dry diethyl ether (200ml). Once the sodium salt had formed, the suspension was cooled to -5° C and phenacyl bromide (20g) in ether (50ml) was added dropwise. The mixture was then allowed to come to room temperature and stirred for a further 16 hours (overnight). The reaction mixture was filtered, the filtrate evaporated and the residue purified on a silica gel column using methylene chloride as eluant. The title compound (22g) was obtained as a white solid of m.p. 40-41° C.

| | | |
|---|---|---|
| Theory for $C_{15}H_{14}OS$<br>Found | C 74.4%<br>C 73.9% | H 5.8%<br>H 5.7% |
| m/e Theory: Found 242:242 | | |

(b) Preparation of 5-methyl-3-phenylbenzo[b]thiophene

Polyphosphoric acid was prepared by adding phosphorus pentoxide (40g) to stirred phosphoric acid (30ml, d:1.68). After the solution had cooled to ca. 70° C, p-tolyl phenacyl sulphide (5g) was added and the mixture heated to 100° C for 2 hours. The reaction mixture was poured onto ice-water and the whole was extracted with diethyl ether. The combined extracts were dried over magnesium sulphate and evaporated. The residual oil was purified on a silica-gel column using hexane as eluant to give the title compound as a colourless oil (3.4g).

| | | |
|---|---|---|
| Theory for $C_{15}H_{12}S$<br>Found | C 80.3%<br>C 80.6% | H 5.3%<br>H 5.6% |
| m/e Theory: Found 224:224 | | |

Example 7

The 5-methyl-3-phenylbenzo[b]thiophene of Example 6(b) was converted into the corresponding dioxide, m.p. 110-111° C, by the method of Example 3.

| | | |
|---|---|---|
| Theory for $C_{15}H_{12}SO_2$<br>Found<br>m/e Theory: Found | C 70.3%<br>C 70.6%<br>256:256 | H 4.7%<br>H 4.6% |

Example 8

Preparation of 6-chloro-3-phenylbenzo[b]thiophene

A mixture of 4-chloro-2-fluorobenzophenone (2.35g), anhydrous potassium carbonate (2.8g) and thioglycolic acid (0.95g) in dry dimethylformamide (50ml) was refluxed for 17 hours. The solvent was removed in vacuo and the residue partitioned between dilute hydrochloric acid and methylene chloride. The aqueous layer was further extracted with methylene chloride, the combined extracts dried over magnesium sulphate and evaporated. The residue was purified by a silica gel column using methylene chloride as eluant to give the title compound (0.9g) as a colourless oil

| | | |
|---|---|---|
| Theory for $C_{14}H_9ClS$ | C 68.7% | H 3.7% |
| Found | C 69.3% | H 3.8% |
| m/e Theory: Found | 244:244 | |

Example 9

Preparation of 2,3-dihydro-3-phenylbenzo[b]thiophene

Sodium metal (18.0g) was added portionwise just below reflux temperature to a stirred solution of 3-phenylbenzo[b]thiophene (3.0g) in ethanol (450ml). After stirring a further 4 hours, the ethanol was removed and the residue partitioned between water and diethyl ether. Further extraction of the aqueous layer was undertaken. The combined extracts were dried, the ether removed and the residual oil purified on a silica gel column using hexane as eluant to give the title compound (0.6g) as a colourless oil.

| | | |
|---|---|---|
| Theory for $C_{14}H_{12}S$ | C 79.2% | H 5.7 % |
| Found | C 79.9% | H 5.9 % |
| m/e Theory: Found | 212:212 | |

Example 10

Preparation of 6-bromo-2,3-dihydro-3-phenylbenzo[b]thiophene

Aluminium chloride (0.35g) was added to a solution of 6-bromobenzo[b]thiophene (0.5g) in dry benzene (15ml). The solution was refluxed for 1 hour, cooled and poured into dilute hydrochloric acid. The aqueous solution was extracted with diethyl ether. The extracts were dried over anhydrous sodium sulphate and evaporated. The residual oil was purified on a silica gel column using hexane as eluant to give the title compound (0.2g) as a pale yellow oil.

| | | |
|---|---|---|
| Theory for $C_{14}H_{11}BrS$ | C 57.7% | H 3.8 % |
| Found | C 56.8% | H 3.6 % |
| m/e Theory: Found | 290:290 | |

Example 11

a) Aluminium chloride (2.0g) was added in portions to a solution of 5-chlorobenzo[b]thiophene (1.7g) in benzene (50ml). After stirring for a further ½ hour, the reaction mixture was poured onto ice/dilute hydrochloric acid mixture and the organic layer separated. The aqueous was further extracted with diethyl ether. The combined extracts were dried and the solvent removed. The residue was stirred with hexane and the insoluble 5-chlorobenzo[b]thiophene dimer removed. Evaporation of the hexane gave a 50:50 mixture (as established by N.m.r.) of 5-chloro-2,3-dihydro-3-phenylbenzo[b]thiophene and 5-chloro-2,3-dihydro-2-phenylbenzo[b]thiophene as a colourless oil (1.3g).

| | | |
|---|---|---|
| Theory for $C_{14}H_{11}ClS$ | C 68.1% | H 4.5 % |
| Found | C 68.0% | H 4.7 % |
| m/e Theory: Found | 246:246 | |

b) Preparation of 5-chloro-2,3-dihydro-3-phenylbenzo[b]thiophene dioxide

To this isomeric mixture (0.7g) in dichloromethane (20ml) was added meta-chloroperoxybenzoic acid (1.2g). After stirring at room temperature for 1½ hours a saturated solution of sodium bicarbonate was added and the organic layer separated. This was dried over anhydrous sodium sulphate, the solvent removed and the residue chromatographed on silica gel using dichloromethane as eluant to give the title

compound as a white solid (0.2g) of m.p. 114-155 ° C.

| | | |
|---|---|---|
| Theory for $C_{14}H_{11}ClO_2S$ | C 60.4% | H 3.9 % |
| Found | C 60.7% | H 3.7 % |
| m/e Theory: Found | 278:278 | |

Examples 12 to 29

By a method analogous to that of Example 1, further compounds of the general formula I were prepared from the corresponding substituted benzoyl chlorides. One compound was then oxidized by a method analogous to that of Example 3, but using half the molar proportion of oxidising agent, to form the corresponding 1-oxide. Details of the compounds thus prepared are given in Table 1.

TABLE 1

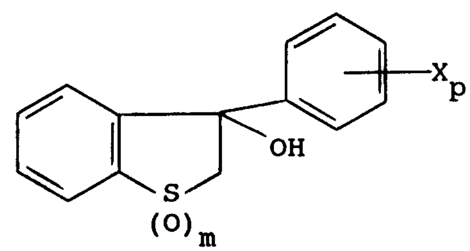

| Example No | In the formula above $X_p$ | m | Elemental Analysis | C | H | m.p. °C | m/e Th/Fd |
|---|---|---|---|---|---|---|---|
| 12 | 4-F | O | Calc. %<br>Found % | 68.3<br>67.8 | 4.5<br>4.5 | oil | 246/246 |
| 13 | 3-F | O | Calc. %<br>Found % | 68.3<br>66.5 | 4.5<br>4.5 | oil | 246/246 |
| 14 | 2-F | O | Calc. %<br>Found % | 68.3<br>69.9 | 4.5<br>4.9 | oil | 246/246 |
| 15 | 3,4 di-F | O | Calc. %<br>Found % | 63.6<br>64.0 | 3.8<br>4.3 | oil | 264/264 |
| 16 | 4-Cl | O | Calc. %<br>Found % | 64.0<br>64.8 | 4.2<br>4.5 | oil | 262/262 |

TABLE 1 (continued)

| Example No | In the formula above $X_p$ | m | Elemental Analysis | C | H | m.p. °C | m/e Th/Fd |
|---|---|---|---|---|---|---|---|
| 17 | 2-Cl | O | Calc. %<br>Found % | 64.0<br>62.5 | 4.2<br>4.1 | oil | 262/262 |
| 18 | 3-Cl | O | Calc. %<br>Found % | 64.0<br>64.5 | 4.2<br>4.4 | oil | 262/262 |
| 19 | 3-OMe | O | Calc. %<br>Found % | 69.8<br>70.8 | 5.4<br>5.6 | oil | 258/258 |
| 20 | 2-Me | O | Calc. %<br>Found % | 74.4<br>78.1 | 5.8<br>6.3 | oil | 242/242 |
| 21 | 4-Me | O | Calc. %<br>Found % | 74.4<br>71.7 | 5.8<br>5.5 | oil | 242/242 |
| 22 | 3-Me | O | Calc. %<br>Found % | 74.4<br>64.8 | 5.8<br>4.0 | oil | 242/242 |
| 23 | 2,6-di-F | O | Calc. %<br>Found % | 63.6<br>64.8 | 3.8<br>4.0 | oil | 264/264 |
| 24 | 2,4-di-F | O | Calc. %<br>Found % | 63.6<br>63.1 | 3.8<br>4.1 | oil | 264/264 |
| 25 | 3,4-di-Cl | O | Calc. %<br>Found % | 56.8<br>56.3 | 3.4<br>3.4 | 92-93 | 296/296 |

**TABLE 1 (continued)**

| Example No | In the formula above $X_p$ | m | Elemental Analysis | C | H | m.p. °C | m/e Th/Fd |
|---|---|---|---|---|---|---|---|
| 26 | 3,5 di-Cl | 0 | Calc. % | 56.8 | 3.4 | oil | 296/296 |
| | | | Found % | 58.0 | 3.9 | | |
| 27 | 2,3 di-Cl | 0 | Calc. % | 56.8 | 3.4 | oil | 296/296 |
| | | | Found % | 57.6 | 3.5 | | |
| 28 | $3-CF_3$ | 0 | Calc. % | 60.8 | 3.7 | oil | 296/296 |
| | | | Found % | 61.9 | 4.3 | | |
| 29 | 2-Cl | 1 | Calc. % | 60.4 | 4.0 | 154-155 | 278/278 |
| | | | Found % | 58.7 | 4.1 | | |

Examples 30 to 63

By methods analogous to those of Examples 2 and 3, further compounds of the general formula I were prepared, compounds of Examples 30 to 61 being prepared from the corresponding compounds of Table 1.

Details of compounds prepared by this route are given in Table 2.

Examples 64 to 72

By a method analogous to that of Example 6(a), intermediates of the general formula V were prepared from the corresponding substituted thiols, and were converted by methods analogous to those of Examples 6(b) and 7 into compounds of the general formula I. Details of compounds of the general formula I prepared by this route are also given in Table 2; details of intermediates prepared are given in Table 3.

Examples 73 to 77

By a method analogous to that of Example 8, further compounds of the general formula I were prepared. One compound was prepared by oxidation of the compound of Example 8 by a method analogous to that of Example 3. Details of compounds prepared by this route are also given in Table 2.

TABLE 2

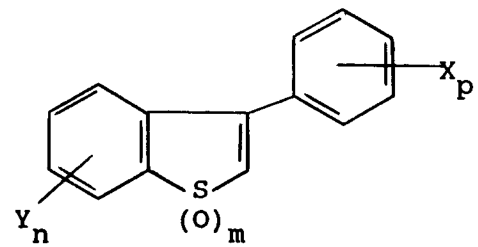

| Example No | $X_p$ | $Y_n$ | m | Elemental Analysis | C | H | m.p. °C | m/e Th/Fd |
|---|---|---|---|---|---|---|---|---|
| 30 | 4-F | - | 0 | Calc. %<br>Found % | 73.7<br>73.9 | 3.9<br>4.2 | oil | 228/228 |
| 31 | 4-F | - | 2 | Calc. %<br>Found % | 64.6<br>64.0 | 3.5<br>3.5 | 160-161 | 260/260 |
| 32 | 3-F | - | 0 | Calc. %<br>Found % | 73.7<br>72.5 | 3.9<br>4.1 | oil | 228/228 |
| 33 | 3-F | - | 2 | Calc. %<br>Found % | 64.6<br>64.4 | 3.5<br>3.6 | 145-146 | 260/260 |
| 34 | 2-F | - | 0 | Calc. %<br>Found % | 73.7<br>74.4 | 3.9<br>4.6 | oil | 228/228 |

(Column headings $X_p$, $Y_n$ and m are grouped under "In the formula above".)

TABLE 2 (continued)

| Example No | In the formula above $X_p$ | $Y_n$ | m | Elemental Analysis | C | H | m.p. °C | m/e Th/Fd |
|---|---|---|---|---|---|---|---|---|
| 35 | 2-F | - | 2 | Calc. %<br>Found % | 64.6<br>63.7 | 3.5<br>3.6 | 141-142 | 260/260 |
| 36 | 3,4 di-F | - | 0 | Calc. %<br>Found % | 68.3<br>70.0 | 3.2<br>3.7 | oil | 246/246 |
| 37 | 3,4 di-F | - | 2 | Calc. %<br>Found % | 60.4<br>60.2 | 2.9<br>3.1 | 143-144 | 278/278 |
| 38 | 4-Cl | - | 0 | Calc. %<br>Found % | 68.7<br>69.5 | 3.7<br>4.2 | 56-57 | 244/244 |
| 39 | 4-Cl | - | 2 | Calc. %<br>Found % | 60.8<br>59.3 | 3.3<br>3.3 | 162-163 | 276/276 |
| 40 | 2-Cl | - | 0 | Calc. %<br>Found % | 68.7<br>68.9 | 3.7<br>3.8 | oil | 244/244 |
| 41 | 2-Cl | - | 2 | Calc. %<br>Found % | 60.8<br>59.0 | 3.3<br>3.4 | 178-179 | 276/276 |
| 42 | 3-Cl | - | 0 | Calc. %<br>Found % | 68.7<br>68.3 | 3.7<br>3.9 | oil | 244/244 |
| 43 | 3-Cl | - | 2 | Calc. %<br>Found % | 60.8<br>60.8 | 3.3<br>3.4 | 115-116 | 276/276 |

TABLE 2 (continued)

| Example No | In the formula above $X_p$ | $Y_n$ | m | Elemental Analysis | C | H | m.p. °C | m/e Th/Fd |
|---|---|---|---|---|---|---|---|---|
| 44 | 3-OMe | - | 0 | Calc. % | 75.0 | 5.0 | oil | 240/240 |
| | | | | Found % | 75.3 | 5.1 | | |
| 45 | 3-OMe | - | 2 | Calc. % | 66.2 | 4.4 | 123-125 | 272/272 |
| | | | | Found % | 67.8 | 4.7 | | |
| 46 | 2-Me | - | 0 | Calc. % | 80.4 | 5.4 | oil | 224/224 |
| | | | | Found % | 80.3 | 5.6 | | |
| 47 | 2-Me | - | 2 | Calc. % | 70.3 | 4.7 | 104-105 | 256/256 |
| | | | | Found % | 69.9 | 4.9 | | |
| 48 | 4-Me | - | 0 | Calc. % | 80.4 | 5.4 | oil | 224/224 |
| | | | | Found % | 80.4 | 5.6 | | |
| 49 | 4-Me | - | 2 | Calc. % | 70.3 | 4.7 | 143-144 | 256/156 |
| | | | | Found % | 68.9 | 5.2 | | |
| 50 | 3-Me | - | 0 | Calc. % | 80.4 | 5.4 | oil | 224/224 |
| | | | | Found % | 81.3 | 5.5 | | |
| 51 | 3-me | - | 2 | Calc. % | 70.3 | 4.7 | 112-113 | 256/256 |
| | | | | Found % | 68.9 | 4.9 | | |
| 52 | 2,6 di-F | - | 0 | Calc. % | 68.3 | 3.3 | oil | 246/246 |
| | | | | Found % | 69.6 | 3.6 | | |

TABLE 2 (continued)

| Example No | In the formula above $X_p$ | $Y_n$ | m | Elemental Analysis | C | H | m.p. °C | m/e Th/Fd |
|---|---|---|---|---|---|---|---|---|
| 53 | 2,6 di-F | - | 2 | Calc. %<br>Found % | 60.4<br>61.0 | 2.9<br>3.0 | 148-149 | 278/278 |
| 54 | 2,4 di-F | - | 0 | Calc. %<br>Found % | 68.3<br>68.7 | 3.3<br>3.5 | 52-53 | 246/246 |
| 55 | 2,4 di-F | - | 2 | Calc. %<br>Found % | 60.4<br>59.8 | 2.9<br>2.6 | 146-147 | 278/278 |
| 56 | 3,4 di-Cl | - | 0 | Calc. %<br>Found % | 60.4<br>61.1 | 2.9<br>3.1 | 52-53 | 278/278 |
| 57 | 3,5 di-Cl | - | 0 | Calc. %<br>Found % | 60.4<br>60.4 | 2.9<br>3.1 | 94-95 | 278/278 |
| 58 | 3,5 di-Cl | - | 2 | Calc. %<br>Found % | 54.2<br>53.4 | 2.6<br>2.8 | 148-150 | 310/310 |
| 59 | 2,3 di-Cl | - | 2 | Calc. %<br>Found % | 54.2<br>53.7 | 2.6<br>2.8 | 153-154 | 310/310 |
| 60 | $3-CF_3$ | - | 0 | Calc. %<br>Found % | 64.7<br>65.2 | 3.2<br>3.5 | oil | 278/278 |
| 61 | $3-CF_3$ | - | 2 | Calc. %<br>Found % | 58.1<br>58.8 | 2.9<br>3.0 | 84-85 | 310/310 |

TABLE 2 (continued)

| Example No | In the formula above $X_p$ | $Y_n$ | m | Elemental Analysis | C | H | m.p. °C | m/e Th/Fd |
|---|---|---|---|---|---|---|---|---|
| 62 | 4-OMe | - | 0 | Calc. %<br>Found % | 75.0<br>74.4 | 5.0<br>5.4 | oil | 240/240 |
| 63 | 4-CN | - | 0 | Calc. %<br>Found % | 76.6<br>75.6 | 3.8<br>4.1 | 101-102 | 235/235 |
| 64 | - | 5-Cl | 0 | Calc. %<br>Found % | 68.7<br>68.9 | 3.7<br>3.7 | 78-79 | 244/244 |
| 65 | - | 5-Cl | 0 | Calc. %<br>Found % | 60.8<br>59.7 | 3.3<br>3.4 | 153-154 | 276/276 |
| 66 | - | 6-Me | 2 | Calc. %<br>Found % | 70.3<br>69.7 | 4.7<br>4.7 | 118-119 | 256/256 |
| 67 | - | 6-Cl | 2 | Calc. %<br>Found % | 60.9<br>60.6 | 3.3<br>3.4 | 142-143 | 276/276 |
| 68 | - | 6,7-di-Cl | 0 | Calc. %<br>Found % | 60.2<br>59.8 | 2.9<br>3.1 | 105-106 | 278/278 |
| 69 | - | 7-Cl | 0 | Calc. %<br>Found % | 68.7<br>68.7 | 3.7<br>3.8 | oil | 244/244 |
| 70 | - | 6-MeO | 0 | Calc. %<br>Found % | 75.0<br>76.1 | 5.0<br>5.0 | oil | 240/240 |

TABLE 2 (continued)

| Example No | In the formula above $X_p$ | $Y_n$ | m | Elemental Analysis | C | H | m.p. °C | m/e Th/Fd |
|---|---|---|---|---|---|---|---|---|
| 71 | - | 6-MeO | 2 | Calc. % | 66.2 | 4.4 | 160-162 | 272/272 |
| | | | | Found % | 68.2 | 4.3 | | |
| 72 | - | 5,6-di-Cl | 0 | Calc. % | 60.2 | 2.9 | 123-124 | 279/279 |
| | | | | Found % | 60.2 | 3.0 | | |
| 73 | - | 6-MeS | 0 | Calc. % | 70.3 | 4.7 | 68-69 | 256/256 |
| | | | | Found % | 70.2 | 4.8 | | |
| 74 | - | 6-$CF_3$ | 0 | Calc. % | 64.7 | 3.2 | oil | 278/278 |
| | | | | Found % | 65.0 | 3.4 | | |
| 75 | - | 6-Cl | 1 | Calc. % | 64.5 | 3.5 | 113-114 | 260/260 |
| | | | | Found % | 62.3 | 3.5 | | |
| 76 | - | 6-PhO | 0 | Calc. % | 79.5 | 4.6 | 77-78 | 302/302 |
| | | | | Found % | 79.4 | 4.6 | | |
| 77 | 4-t-Bu | 6-Cl | 0 | Calc. % | 72.0 | 5.7 | oil | 300/300 |
| | | | | Found % | 72.5 | 6.0 | | |

TABLE 3

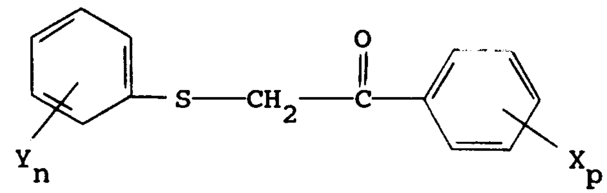

| Intermediate No | In the formula above $X_p$ | $Y_n$ | Elemental Analysis | C | H | m.p. °C | m/e Th/Fd |
|---|---|---|---|---|---|---|---|
| A | - | 4-Cl | Calc. %<br>Found % | 64.1<br>64.1 | 4.2<br>4.3 | 83-84 | 262/262 |
| B | - | 3-me | Calc. %<br>Found % | 74.4<br>74.5 | 5.8<br>5.9 | oil | 242/242 |
| C | - | 3-Cl | Calc. %<br>Found % | 64.1<br>64.1 | 4.2<br>4.2 | 66-67 | 262/262 |
| D | - | 2,3 di-Cl | Calc. %<br>Found % | 56.6<br>63.8 | 3.4<br>4.3 | 117-118 | 296/296 |
| E | - | 2-Cl | Calc. %<br>Found % | 64.1<br>63.8 | 4.2<br>4.3 | 91-92 | 262/262 |
| F | - | 3-MeO | Calc. %<br>Found % | 69.7<br>69.6 | 5.4<br>5.3 | 44-45 | 258/258 |

TABLE 3

| Intermediate No | In the formula above | | Elemental Analysis | | | m.p. °C | m/e Th/Fd |
|---|---|---|---|---|---|---|---|
| | $X_p$ | $Y_n$ | | C | H | | |
| G | - | 3,4-di-Cl | Calc. % | 56.6 | 3.4 | 75-77 | 296-296 |
| | | | Found % | 56.4 | 3.5 | | |

Example 78

Acaricidal Activity (ovicide)

The acaricidal activity of various compounds of the general formula I was assessed, employing eggs of the glazzhouze red spider mite, Tetranychus urticae (T.u.), less than 24 hours old, by the following procedure.

Solutions or suspensions of test compound were first made up over a range of concentrations in water (initially 0.1% by weight) containing 10% by weight acetone and 0.025% by weight "Triton X-100" ("Triton" is a trade mark) surface active agent (the condensation product of ethylene oxide with an alkylphenol).

2cm diameter leaf discs cut from the leaves of French bean plants were placed on filter paper, kept moist by a cotton wool wick dipped into water.

On the day before spraying, each leaf disc was infested with 10 female adult mites. On the day of the test, the adults were removed, leaving the eggs laid overnight on the discs. The leaf discs were then sprayed with solutions of test compound made up as described above, at a rate equivalent to 340 litres per hectare (3.4 x 10-5 m3/m2).

Throughout the test, the eggs were held under normal conditions (23 ° C ± 2 ° C, fluctuating humidity and 16 hours day length). After 7 to 10 days, the numbers of hatched nymphs and unhatched eggs were assessed and the percentage mortality calculated. The results are given in Table 4.

TABLE 4

Ovicidal activity

| Compound of Example No. | Mortality Grade |
|---|---|
| 1 | A |
| 2 | A |
| 3 | A |
| 5 | A |
| 6 | A |
| 7 | A |
| 8 | A |
| 9 | A |
| 10 | A |
| 11 | B |
| 12 | A |
| 13 | A |
| 14 | A |
| 15 | A |
| 16 | A |
| 17 | A |
| 18 | A |
| 19 | C |
| 20 | A |
| 21 | C |
| 22 | A |
| 23 | A |
| 24 | A |
| 25 | A |
| 30 | A |
| 31 | A |
| 32 | A |
| 33 | A |
| 34 | A |
| 35 | A |
| 36 | A |

TABLE 4 (continued)

Ovicidal activity

| Compound of Example No. | Mortality Grade |
|---|---|
| 37 | A |
| 38 | A |
| 39 | A |
| 40 | A |
| 41 | A |
| 42 | A |
| 43 | A |
| 44 | A |
| 45 | C |
| 46 | A |
| 47 | A |
| 48 | B |
| 49 | C |
| 50 | B |
| 51 | C |
| 52 | A |
| 53 | A |
| 54 | A |
| 55 | A |
| 56 | A |
| 57 | A |
| 58 | C |
| 59 | A |
| 60 | A |
| 61 | A |
| 62 | C |
| 63 | A |
| 64 | A |
| 65 | A |
| 66 | A |
| 67 | A |

TABLE 4 (continued)

Ovicidal activity

| Compound of Example No. | Mortality Grade |
|---|---|
| 68 | A |
| 69 | C |
| 70 | A |
| 71 | A |
| 72 | A |
| 73 | A |
| 74 | A |
| 75 | A |
| 76 | A |
| 77 | A |

Grades A, B and C indicate mortalities of 70-100%, 40-69% and up to 39%, respectively, at the initial test concentration of 0.1% by weight (1000 ppm).

Example 79

Acaricidal Activity (mite life cycle)

The acaricidal activity of various compounds of the general formula I was assessed, employing adult female glasshouse red spider mites, Tetranychus urticae (T.u.), by the following procedure.

2cm diameter leaf discs cut from the leaves of French bean plants were placed, underside uppermost, on 5.5cm diameter filter papers, kept moist by a cotton wool wick dipped in water.

Each leaf disc was infested with 25 to 30 adult female mites which were removed after 6 hours, leaving about 50 eggs on each disc. Within 5 days the eggs hatched. The freshly emerged larvae on the leaf discs were sprayed with solutions of test compound made up as in Example 78 above, at a rate equivalent to 340 litres per hectare (3.4 x 10-5 m3/m2).

The discs were thereafter kept under normal laboratory conditions (23°C ± 2°C), fluctuating humidity and 16 hours day length). After 7 days assessment was made of the number of mites emerging as adults.

From the results, the dosage of active ingredient required to kill half of the test species was calculated for each test compound and was graded as follows:

% active ingredient in spray :

<0.1 and >0.01 +

<0.01 and >0.001 + +

<0.001 + + +

The results are given in Table 5.

TABLE 5

Acaricidal Activity

| Compound of Example Example No. | Grade |
|---|---|
| 2 | +++ |
| 3 | +++ |
| 5 | + |
| 8 | +++ |
| 12 | +++ |
| 14 | + |
| 15 | + |
| 16 | ++ |
| 17 | ++ |
| 18 | +++ |

TABLE 5 (continued)
Acaricidal Activity

| Compound of Example Example No. | Grade |
|---|---|
| 22 | + |
| 23 | + |
| 24 | + |
| 30 | +++ |
| 32 | ++ |
| 35 | +++ |
| 36 | + |
| 37 | ++ |
| 38 | ++ |
| 39 | ++ |
| 40 | ++ |
| 41 | ++ |
| 42 | +++ |
| 43 | +++ |
| 52 | + |
| 53 | +++ |
| 54 | +++ |
| 55 | +++ |
| 61 | ++ |
| 67 | +++ |
| 68 | ++ |
| 71 | + |
| 72 | + |
| 73 | + |
| 74 | + |
| 75 | +++ |
| 76 | + |

## Claims

1. A pesticidal composition which comprises a compound of the general formula

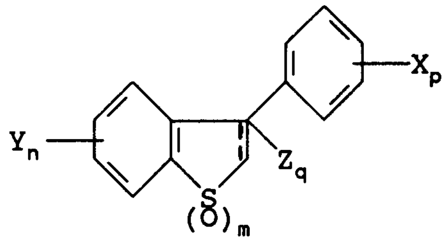

I

in which

X and Y each represents a halogen atom or an alkyl, haloalkyl, alkoxy, haloalkoxy, alkylenedioxy, alkylthio, halo-substituted alkylthio, alkylsulphinyl, halo-substituted alkylsulphinyl, alkylsulphonyl, halo-substituted alkylsulphonyl, cyano, nitro, amino, alkylamino, dialkylamino, acetamido, N-alkyl-acetamido, optionally substituted benzyl, optionally substituted benzoyl, optionally substituted phenylthio, optionally substituted phenylsulphinyl, optionally substituted phenylsulphonyl or optionally substituted phenoxy radical,

p represents 0 or an integer of from 1 to 5,

n represents 0 or an integer of from 1 to 4, any two or more substituents represented by X and/or Y may be the same or different,

m represents 0, 1 or 2,

Z represents a hydrogen atom, a hydroxy group or a radical OR where R represents an acyl moiety, q represents 0 or 1, and

¦| represents a single bond (when q is 1) or a double bond (when q is 0),

provided that if q represents 1 and Z represents a hydroxy group or an OR radical, then n = 0 and p = 0, or when n ≧ 1, then p = 0, or when p ≧ 1, then n = 0,

together with a suitable carrier.

2. A composition as claimed in claim 1, wherein p represents 0, 1 or 2 and substituent(s) represented by X, if present, are selected from fluorine and chlorine atoms and methyl, methoxy, cyano and trifluoromethyl groups.

3. A composition as claimed in claim 1, wherein n represents 0, 1 or 2 and substituent(s) represented by Y, if present, are selected from chlorine atoms and methyl and methoxy groups.

4. A composition as claimed in any one of claims 1 to 3, which comprises at least two carriers, at least one of which is a surface active agent.

5. A method of combating pests at a locus, which comprises applying to the locus a compound of the general formula I as specified in any one of claims 1 to 3, or a pesticidal composition as claimed in any one of claims 1 to 4.

6. The use of a compound of the general formula I as specified in any one of claims 1 to 3, as a pesticide, particularly as an acaricide.

7. A compound of the general formula I as specified in claim 1, wherein

- if q = 0 and ¦| represents a double bond, then n + p ≧ 1; if, also, m = 0, then
  - $X_p$ is not a bromine atom or a methoxy group or 2-methylthio when n = 0,
  - $Y_n$ is not 5-bromo, 5-chloro, 6- or 7-methoxy, 7-methyl, 5,7-dimethyl or 4,7-dimethoxy when p = 0, and
  - $X_p$ is not 4-bromo when $Y_n$ is 5-methyl, nor any 4-halo when $Y_n$ is 7-methyl; if, also, m = 1, then
  - $X_p$ is not 4-chloro when $Y_n$ is 2,6-dichloro;
    and if, also, m = 2, then
  - $Y_n$ is not 2-methyl;
- if q = 1, ¦| represents a single bond and Z represents H, and if, also, m = 0 or 2,
  - when n = 0, then
    p ≧ 1 and additionally
    $X_p$ is not 4-methyl or (when m = 2) 2,5- or 3,4- dimethyl, and

- when p = 0, then
  n ≧ 1 and additionally
  $Y_n$ is not 5-methyl, 5-ethyl or 5,7-dimethyl; and
- if q = 1, ¦| represents a single bond and Z represents OH, then
  - p ≧ 1 when m = 0 or 2, and
  - X is not 4-methyl when, simultaneously, m = 0 and p = 1.

**8.** A process for the preparation of a compound as claimed in claim 7, which comprises
(a) reacting thioanisole of the formula

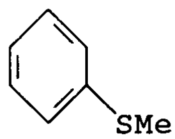

II

with an alkyl lithium compound and an optionally substituted benzoyl halide of the general formula

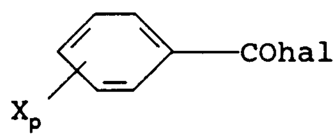

III

in which X and p have the meanings given in claim 1, to form a compound of the general formula

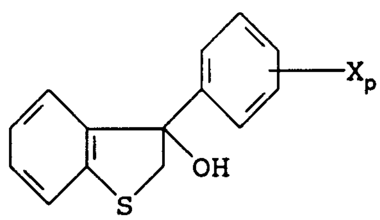

$Ic_1$

in which X and p have the meanings given in claim 1,
(b) cyclising a compound of the general formula

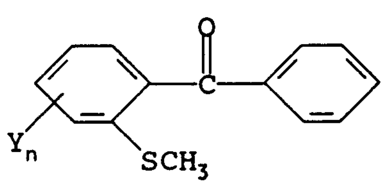

IV

in which Y and n have the meanings given in claim 1, in the presence of a base and a solvent, to form a compound of of the general formula

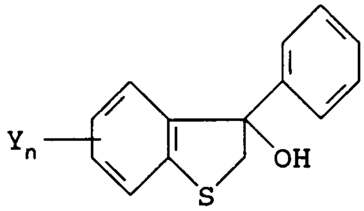

Id$_1$

in which Y and n have the meanings given in claim 1, or

(c)

(i) dehydrating a compound of the general formula Ic$_1$ given above to give a compound of the general formula

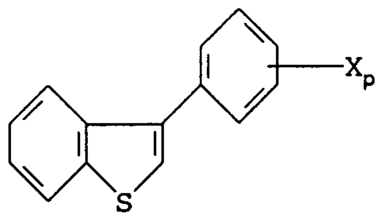

Ia$_1$

in which X and p have the meanings given in claim 1, or dehydrating a compound of the general formula Id$_1$ given above to give a compound of the general formula

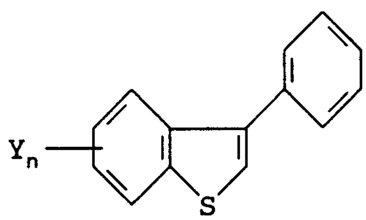

Ia$_3$

in which Y and n have the meanings given in claim 1,

or

ii) cyclising a compound of the general formula

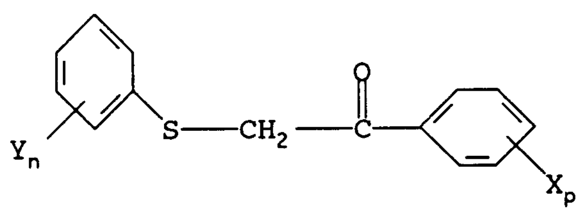

V

in which X, Y, p and n have the meaning given in claim 1, in the presence of a dehydrating agent, or reacting a compound of the general formula

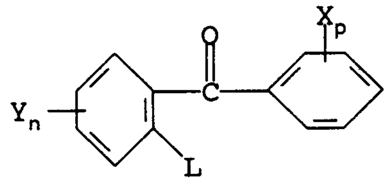

VI

in which X, Y, p and n have the meanings given in claim 1 and L represents a leaving group, with thioglycolic acid in the presence of a base and a solvent, to form a compound of the general formula

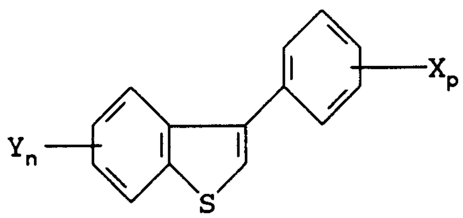

$Ia_4$

in which X, Y, p and n have the meanings given in claim 1, or

(d)

(i) reacting a compound of the general formula

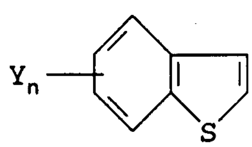

IX

in which Y and n have the meanings given in claim 1, with a compound of the general formula

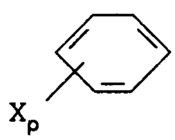

X

in which X and p have the meanings given in claim 1, in the presence of aluminium chloride, and separating the desired isomer, if required, of the general formula

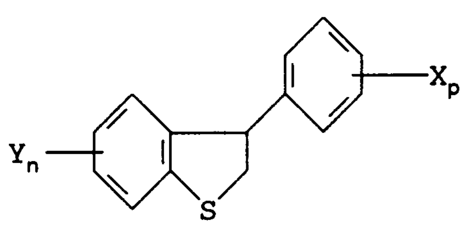

$Ib_1$

in which X, Y, p and n have the meanings given in claim 1, or

(ii) reducing a compound of the general formula $Ia_4$ above to form a compound of the general formula $Ib_1$ above,

and, if desired, converting a resulting compound of the general formula I into another such compound, for example by carrying out one or more of the following reactions, where appropriate in any desired order: acylating a 3-hydroxy group, oxidising a thiophene to a 1-oxide or 1,1-dioxide.

**9.** A process as claimed in claim 8, wherein the compound of the general formula V is prepared by reaction of a compound of the general formula

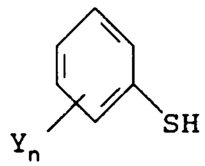

VII

in which Y and n have the meanings given in claim 1, in the presence of a suitable base with an optionally substituted phenacyl halide of the general formula

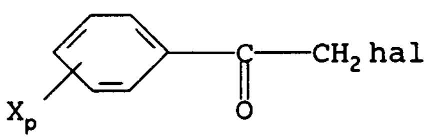

VIII

in which X and p have the meanings given in claim 1.

**10.** A compound of the general formula

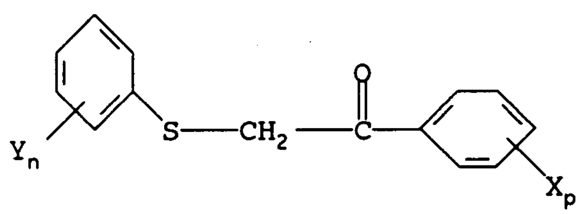

V

in which X, Y, p and n have the meanings given in claim 1.

European Patent Office

# EUROPEAN SEARCH REPORT

Application Number

EP 92 20 2438
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X<br><br><br><br><br><br><br>A | TETRAHEDRON LETTERS.<br>vol. 27, no. 38, 1986, OXFORD GB<br>pages 4625 - 4628<br>S. CABIDDU ET AL. 'Metalation Reactions. IX. Dilithiation of Aromatic Thioethers'<br>* the whole document, and in particular compound no. 11 *<br>--- | 10<br><br><br><br><br><br><br>8 | C07D333/54<br>C07D333/64<br>C07C323/22<br>A01N43/12 |
| X | CHEMICAL ABSTRACTS, vol. 108, no. 9, 29 February 1988, Columbus, Ohio, US;<br>abstract no. 75148m,<br>T. HIROTA ET AL. 'A novel synthesis of benzofuran and related compounds. IV. The Vilsmeier reaction of 3,5-dimethoxyphenylthiomethyl compounds'<br>page 657 ;column 1 ;<br>* abstract and RN [112664-32-1] *<br>& HETEROCYCLES<br>vol. 26, no. 10, 1987,<br>pages 2717 - 2725<br>--- | 10 | |
| X | CHEMICAL ABSTRACTS, vol. 111, no. 15, 9 October 1989, Columbus, Ohio, US;<br>abstract no. 133622m,<br>H. ISHIBASHI ET AL. 'A convenient general access to alpha-sulfenylated acetophenones and alkanones'<br>page 708 ;column 1 ;<br>* abstract and RN's [101169-29-3], [122657-57-2] *<br>& SYNTH. SOMMUN.<br>vol. 19, no. 3-4, 1989,<br>pages 443 - 452<br>---<br>-/-- | 10 | TECHNICAL FIELDS SEARCHED (Int. Cl.5)<br>C07D<br>C07C |

The present search report has been drawn up for all claims

| Place of search | Date of completion of the search | Examiner |
|---|---|---|
| THE HAGUE | 27 OCTOBER 1992 | B. Paisdor |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 92 20 2438
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 96, no. 1, 5 July 1982, Columbus, Ohio, US; abstract no. 85357b, P. D. CLARK ET AL. 'Addition reactions of benzo[b]thiophen. Part 2. Reactions of substituted benzo[b]thiophenes in benzene or toluene' page 571 ;column 1 ; * abstract and RN's [80824-40-4], [80824-37-9], [80824-32-4], [80824-29-9] * & J. CHEM. RES., SYNOP. no. 10, 1981, page 307 | 7 | |
| X | JOURNAL OF THE CHEMICAL SOCIETY, PERKIN TRANSACTIONS 1. no. 2, 1982, LETCHWORTH GB pages 615 - 621 P. D. CLARK ET AL. 'Addition Reactions of Benzo[b]thiophen. Part 3. Additon and Ring-opening Reactions with Phenolic Ethers' * the whole document * | 7 | |
| Y | | 7,8,10 | TECHNICAL FIELDS SEARCHED (Int. Cl.5) |
| X | JOURNAL OF THE CHEMICAL SOCIETY, PERKIN TRANSACTIONS 1. no. 3, 1980, LETCHWORTH GB pages 677 - 685 P. D. CLARK ET AL. 'Addition Reactions of Benzo[b]thiophen. Part 1. Self-addition and Addition of Simple Aromatic Hydrocarbons' * the whole document * | 7 | |
| Y | | 7,8,10 | |

-/--

The present search report has been drawn up for all claims

| Place of search | Date of completion of the search | Examiner |
|---|---|---|
| THE HAGUE | 27 OCTOBER 1992 | B. Paisdor |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 92 20 2438
Page 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | SYNTHESIS. no. 11, November 1988, STUTTGART DE pages 888 - 890 S. CABIDDU ET AL. 'Metalation Reactions; Part XI. A Novel, One-Step Synthesis of Benzo[b]thiophene Derivatives' * the whole document * | 7,8,10 | |
| A | GB-A-1 242 970 (PARKE, DAVIS & CO.) * the whole document * | 7,8,10 | |
| D,A | US-A-3 433 874 (E. J. GEERING ET AL.) * the whole document * | 1 | |

TECHNICAL FIELDS SEARCHED (Int. Cl.5)

The present search report has been drawn up for all claims

| Place of search | Date of completion of the search | Examiner |
|---|---|---|
| THE HAGUE | 27 OCTOBER 1992 | B. Paisdor |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)